# EUROPEAN PATENT SPECIFICATION

(11) **EP 2 799 103 B1**
(45) Date of publication and mention of the grant of the patent: **13.04.2016**
(21) Application number: 14150364.9
(22) Date of filing: 03.02.2003
(51) Int. Cl.: A61M 16/06

(54) **Breathing assistance apparatus**
Gerät zur Unterstützung der Atmung
Dispositif d'assistance respiratoire

(30) Priority: 04.02.2002 NZ 51703102
(43) Date of publication of application: 05.11.2014
(62) Divisional of application: 03715867.2
(73) Proprietor: Fisher & Paykel Healthcare Limited, 1741 East Tamaki, Auckland (NZ)
(72) Inventor: Mackie, Scott Robert, Auckland 2013 (NZ); White, Craig Karl, 1005 Auckland (NZ); Foreman, Mark, Auckland 2013 (NZ)
(74) Representative: Hoarton, Lloyd Douglas Charles

(56) References cited:
- WO-A1-01/97892
- DE-C- 28 036
- US-A- 3 513 844
- US-A- 3 566 862
- US-A- 5 558 084

## Description

### FIELD OF INVENTION

The present invention relates particularly, though not solely, to the delivery of high flow humidified pressurised oxygen and/or air by way of a wide bore nasal cannula to a patient in need of respiratory support.

### SUMMARY OF THE PRIOR ART

There are two common methods for delivering oxygen to a patient. The simplest method is via a Venturi mask. This method has the advantage of delivering, precisely and constantly, the desired level of oxygen, or fraction of inspired oxygen (FiO2), provided that the FiO₂ is less than 50%. The operation of a Venturi mask is based on the Bernoulli principle. One hundred per cent oxygen flowing through the narrow orifice results in a high-velocity stream that entrains room air through multiple open side ports at the base of the mask or on top a humidifier. The amount of room air entrained to dilute the oxygen depends on the orifice size. Venturi masks can provide FiO₂ levels from 24-50% with great accuracy.

The other method of oxygen delivery is dual prong nasal cannula. Nasal cannula generally consist of an entry tubing, either symmetric or single sided that lies across the upper lip. Protruding from this tubing are open ended prongs which extend into the nares of the patient to deliver oxygen, eg.see US-A-3513844. Nasal cannula have the advantage of being more comfortable and acceptable than a face mask to most patients. When using nasal cannula, however, FiO₂ cannot be precisely controlled because it is affected by the route of inhalation (nose or mouth), upper airway geometry and breathing pattern.

Other, less popular, methods for oxygen delivery are used when a FiO₂ higher than 50% is required. A non-rebreathing mask with reservoir and one-way valve may deliver a FiO₂ of up to 90%, provided that leaks around the mask have been eliminated by tight seals. These masks are rarely used because they are not easily accepted by patients and carry significant risk of the patient rebreathing expired air. The rebreathing of expired air is undesirable as this air contains high levels of carbon dioxide.

As oxygen is supplied as a dry gas it is well known in the art to either heat and/or humidify gases before delivering them for breathing by a patient. In particular when delivering oxygen, or oxygen / air mixture, it has proven beneficial to humidify the gases first. In WO 01/41854 of Vapotherm, Inc. a system is disclosed that allows the delivery of humidified oxygen through a nasal cannula. This system uses a narrow bore conduit and nasal cannula with a high resistance to gas flows, thereby requiring the oxygen be of a high pressure. Air, as well as oxygen, can also be passed down the conduit and nasal cannula and it too must be of a high pressure. This system allows the delivery of high flows of oxygen enriched air to the patient, but is limited in the flows achievable due to the narrow bore of the cannula resulting in high resistance gas flow and excessive velocity and noise upon exiting the cannula. Furthermore, the narrowness of the nasal cannula in this system allows easy expiration of gases between the prongs and nares and therefore does not create any positive airway pressure.

Innomed Technologies, Inc. manufactures a nasal cannula device called the NASALAIRE™. In this device air or oxygen travels down a wide bore conduit to nasal cannula. The NASALAIRE™ creates a physical seal between the nares and itself, and relies on the absence of leaks around itself and the nares to deliver pressure supplied by a continuous positive airway pressure (CPAP) blower to the airway of the wearer. The wearer is required to breathe out and in of the NASALAIRE™, thereby rebreathing some of the exhaled air from the lungs. The NASALAIRE™ is not designed to deliver humidified gases to the patient, being unheated and without insulation of any type.

### SUMMARY OF THE INVENTION

It is an object of the present invention to provide a breathing assistance apparatus which goes someway to overcoming the above mentioned disadvantages or which will at least provide the public a useful choice. The invention is defined by the appended claims.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

One preferred form of the present invention will now be described with reference to the accompanying drawings in which;
**Figure 1** is an illustration of a respiratory humidifier system that may be used with the nasal cannula of the present invention,
**Figure 2** is an illustration of the humidifier base of the respiratory humidifier system of Figure 1,
**Figure 3** is a side view of the nasal cannula of the present invention in use by a patient,
**Figure 4** is a front perspective view of the nasal cannula of the present invention,
**Figure 5** is a back perspective view of the nasal cannula of the present invention,
**Figure 6** is a further back perspective view of the nasal cannula of the present invention, and
**Figure 7** is a side perspective view of a further embodiment of the nasal cannula of the present invention.
**Figure 8** is a side view of nasal prongs showing hidden details and one embodiment of a cannula with pressure relief valve.
**Figure 9** is a cross-sectional side view of the pressure relief valve as shown in Figure 8 when in operation.

### DETAILED DESCRIPTION OF THE PREFERRED EMBODIMENTS

Whether used in a hospital environment or in a home environment, the nasal cannula of the present invention will generally have associated three main pieces of apparatus. Firstly an active humidifier that controls the temperature of a heater plate heating a body of water to achieve a desired temperature and humidity of the gases being humidified. Secondly a transport conduit from the humidifier to the patient is also required, which is preferably heated to reduce condensation, or "rain out". Thirdly a cannula designed to fit up into the nasal cavity and deliver humidified, pressurized gases.

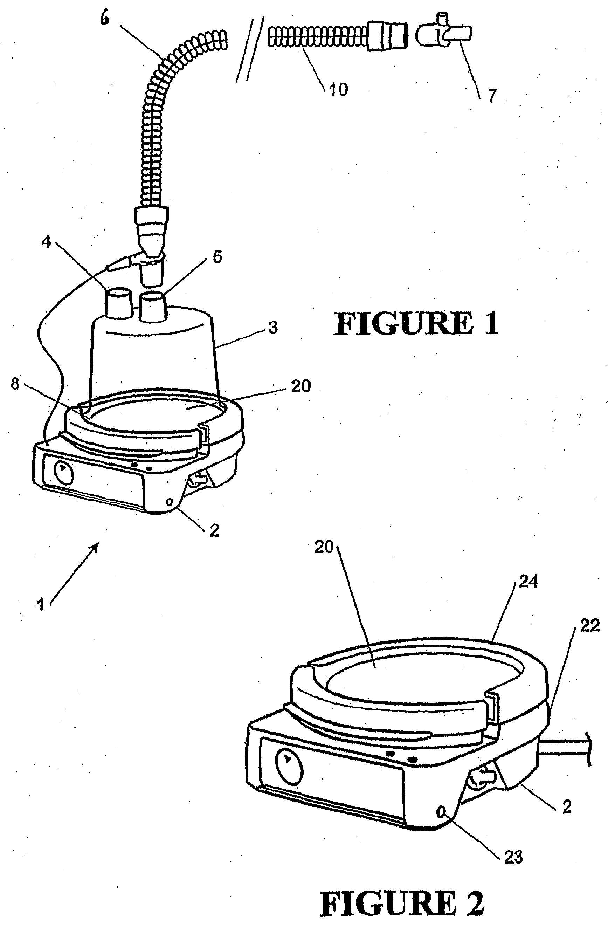

Referring to Figure 1 a humidifying apparatus as might be used in a hospital generally referenced 1 is shown. The apparatus comprises a body 2 containing heating means comprising a heating plate 20 having an electric heating element therein or in thermal contact therewith and control means for example electronic circuitry which may include a microprocessor for controlling the supply of energy to the heating element. The body 2 is removably engageable with a humidifying chamber 3 that contains water for humidifying gases.

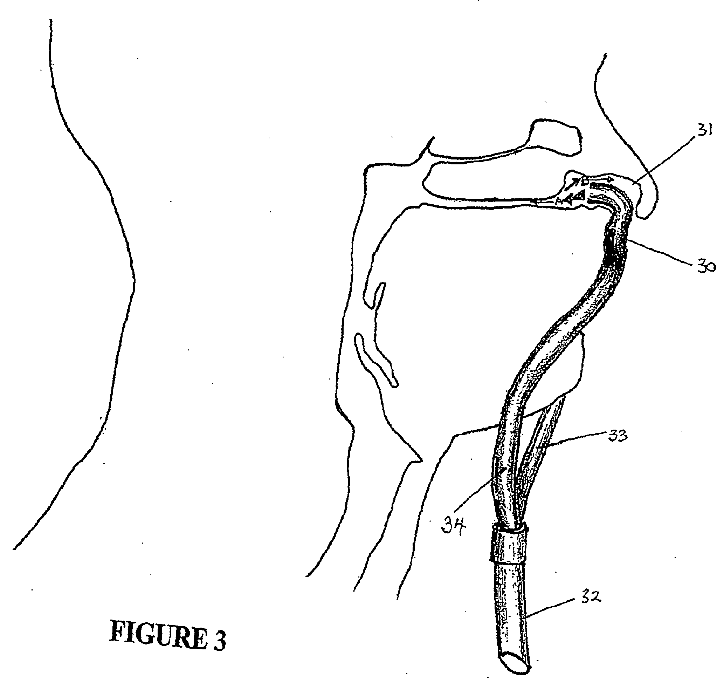

Referring to Figure 2, which shows the humidifier apparatus in more detail, the humidifying chamber 3 has edges, which engage with collar 24 on the humidifier apparatus. The gases to be humidified may be a mixture of air, oxygen and anaesthetic for example, which are supplied to the chamber through gas inlet 4. This might be connected to a ventilator, source of pressurised oxygen, flow generator, or air compressor. A gases outlet 5 is also provided and the gases outlet 5 is connected to the conduit 6 (Figure 1), which conveys humidified gases to the patient at the end 7 of the conduit. The end 7 of the conduit has a cannula (30, in Figure 3) connected to the patients nose so as to supply humidified gases to the user. The humidifier heater plate 20 has a temperature transducer 8 that is in electrical connection with the electronic control circuitry in body 2 of the apparatus so that the control means monitors the temperature of the heating plate.

A heating element 10 is provided within the conduit 6 to help prevent condensation of the humidified gases within the conduit. Such condensation is due to the temperature of the walls of the conduit being close to the ambient temperature, (being the temperature of the surrounding atmosphere) which is usually lower than the temperature of the humidified gases within the conduit. The heater element is effectively replaces the energy lost from the gases through conduction and convection during transit through the conduit. Thus the conduit heater element ensures the gases delivered are at an optimal temperature and humidity.

### Nasal Cannula

The nasal cannula 30 of the present invention is shown in side view in Figure 3 when located within a patient's nares 31. One end of the inlet tube 32 is attached to end 7 of the conduit 6 (see Figure 1) and accepts the gases flow from the pressurised source supplying gases and the humidifier. The other end of the tube 32 terminates at a y-connection where each of the arms of the Y are tubes 32, 33. These tubes connect to the two nasal prongs of the cannula. The diameter of the inlet tube 32 and tubes 33, 34 are as large as possible to ensure minimal pressure drop in the gases before delivery to the patient.

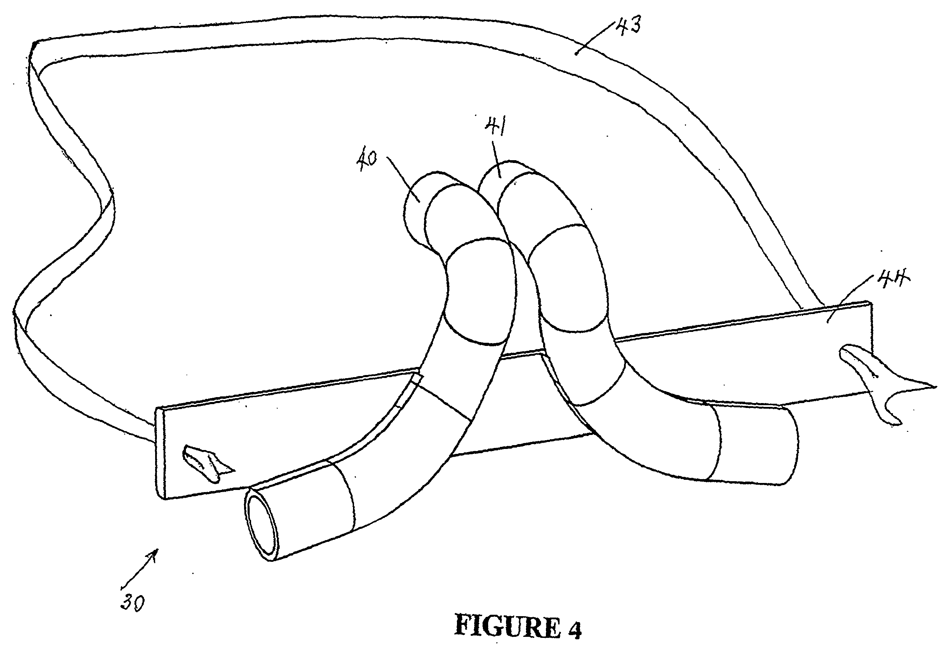

Referring to Figures 3 to 6, operatively attached to a body 44 are two nasal prongs 40, 41. Gases flow from the inlet tube 32 that branches, at a y-connection, into two tubes 32, 33 and these tubes are operatively attached to the nasal prongs 40, 41. Each of the prongs 40, 41 extend into a corresponding nare of the patient. The prongs 40, 41 and body 44 are attached to the patient's face via a strap 43. The strap 43 is attached to the body by appropriate fastening means, for example, VELCRO™, which may be adjusted to suit the size of the patients head and ensures the prongs 40, 41 remain within the patient's nares and body 44 fits comfortably against the patient's face.

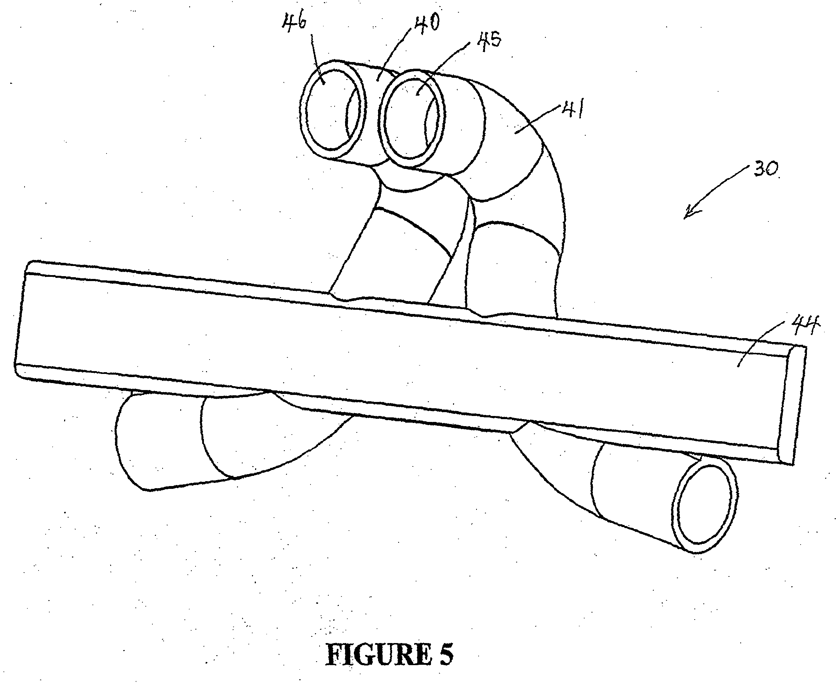

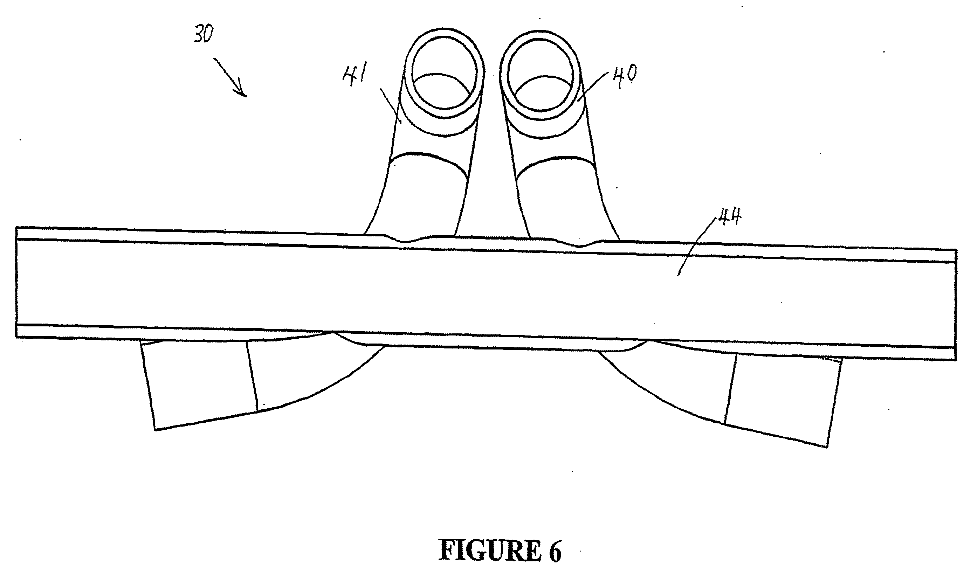

Referring to Figures 5 and 6, the prongs 40, 41 are angled inwards toward the septum of the nose meaning the prongs are anatomically correct and follow the nasal cavity towards the posterior (as can also be seen in Figure 3).

The prongs 40, 41 themselves are round or elliptical in shape or may be shaped to correspond to the inner dimensions of the nasal cavity. The diameter of each of the cannula is chosen such that exhaled gases can be breathed out by the patient past the each of the prongs, as indicated by arrows A and B in Figure 3 , where arrow A shows the direction of inhaled gases and arrow B shows the direction of exhaled gases.

In the embodiment where the prongs 40, 41 follow the inner shape of the patient's nostrils, a more efficient flow of gases into the patient's lungs is achieved. With the cannula shaped in this manner, gases flows are directed down the main nasal passage more accurately. Particularly, each of the prongs 40, 41 are curved towards the sagittal (midline) plane, that is, towards the septum of the nose. Furthermore, the top 45, 46 of each cannula points, in use, toward the back of the patient's head.

The entire assembly of the nasal cannula, including the cannula, body and prongs attaching the cannula to the patient's face and nares, are moulded from silicone or other flexible material as are known is the art for cannula construction.

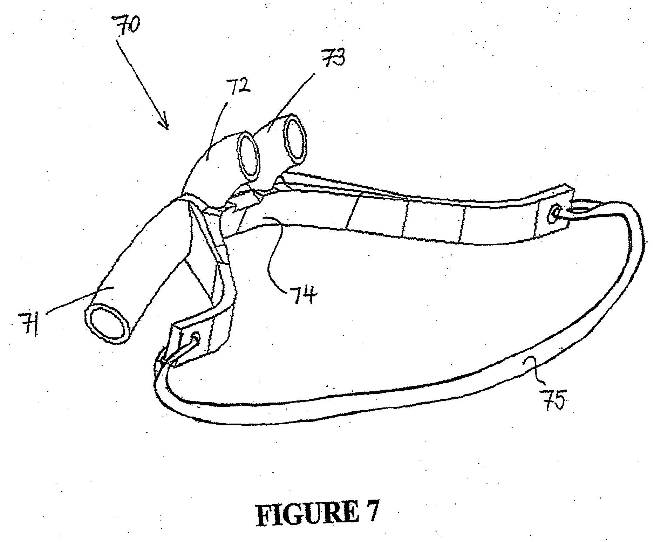

In a further embodiment of the present invention, as shown in Figure 7, the inlet to the cannula 70 is provided through one tube. Here, gases flow from the inlet tube 71 through each of the prongs 72, 73 and into the corresponding nares of the patient. The cannula of this embodiment is also attached to a body section 74 that is shaped so it fits the contours of the patient's face. The cannula and body is maintained against the patients face again by way of a head strap 75.

The cannula of the present invention according to one embodiment comprises wide bore cannula to minimise the flow resistance and the entry velocity. For adults an approximate nare diameter of 6mm has been found to be suitable (actual diameter depends on cannula shape), this compares with 2-3mm in the prior art. This allows the present invention to deliver higher than 30 L/min of oxygen enriched gases, whereas prior art systems with small bore cannula can only deliver an absolute maximum of 6 Umin of dry gas or 20 L/min humidified gas, for the following reasons:
a) Flows higher than 20L/min through existing cannula (i.e. VAPOTHERM™ system) are noisy due to the creation of turbulent gas flow.
b) Existing cannula (i.e. VAPOTHERM™ system) have a high resistance to gas flow, requiring the use of a supply of gas exceeding 50cmH2O pressure.
c) Flows higher than 20L/min through existing narrow bore cannula (i.e. VAPOTHERM™ system) create a jet of gas upon exiting the cannula that becomes irritating to the airway over short periods of time.

The expiration of gases by the patient against the high incoming flow provides positive end expiratory pressure (PEEP). PEEP keeps the airways and alveoli from collapsing at end-expiration and can reopen airways and alveoli that have already collapsed. This improves gas exchange (decreased intra pulmonary shunt), reduces the resistance to airflow (lung resistance), and makes the lungs less stiff (increased lung compliance). Levels of oxygen and carbon dioxide may improve, reducing the need for supplemental oxygen and the sensation of breathlessness. PEEP may also improve cardiac performance by increasing mean intra thoracic pressure. PEEP is of special advantage to assisting in the treatment of obstructive lung diseases and heart failure, including emphysema, bronchiectasis, chronic bronchitis, cystic fibrosis and pulmonary edema.

The wide bore cannula of the present invention also allows for the provision of gases to the patient that exceeds the patient's peak inspiratory flow. Consequently, a small amount of positive pressure is also generated during the inspiratory phase. This will create inspiratory positive airway pressure (IPAP) that, like PEEP, keeps airways and alveoli from collapsing and reduces the effort to inhale. IPAP is of special advantage to patients who experience breathlessness during respiratory failure.

The ability of the cannula of the present invention to provide these forms of pressure support also allows the cannula to deliver pressure oscillations to the patient. Pressure oscillations are known to improve the clearance of sputum from the lungs and the exchange of respiratory gases between the blood and alveolar air.

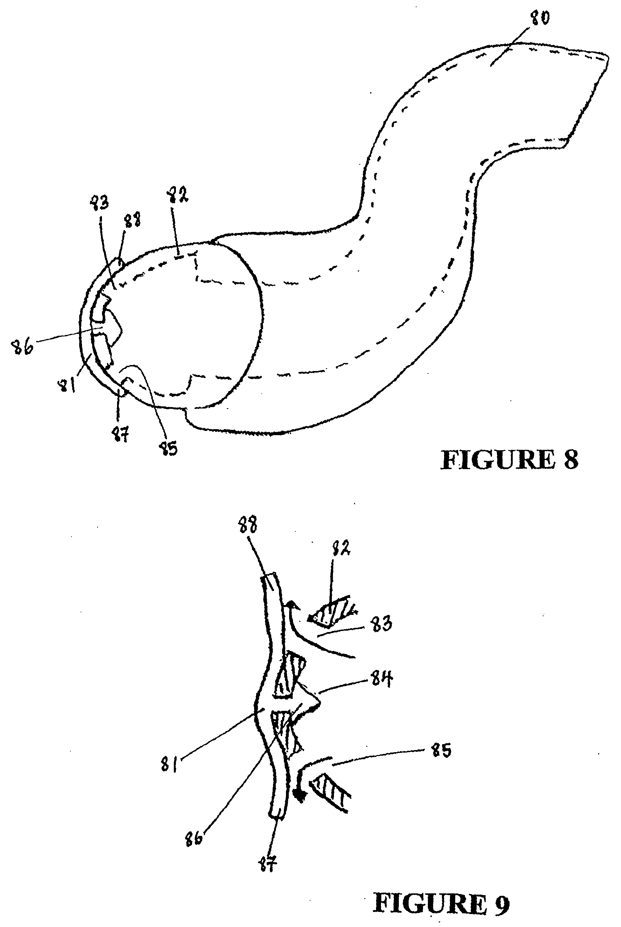

The cannula of the present invention does not create a seal against the nares, and so allow a continuous leakage of gas out of the nose between the cannula and nares. In the event of improper fitting causing the prongs to seal against the nares, one embodiment of the nasal cannula of the present invention is provided with a pressure relief valve. This valve will ensure barotrauma is not inflicted upon the patient. This pressure relief could be similar in form to a CPAP valve as is known in the art or could be constructed integrally within the moulding of the nasal cannula as shown in Figure 8. The continuous flow around the nares allowed by the device not sealing also eliminates the need for a bias flow outlet such as that incorporated in the NASAL-AIRE™ manufactured by Innomed Inc.

Referring now to Figures 8 and 9, one embodiment of nasal cannula with prongs 80 is shown (without additional head straps and the like) that has a pressure relief valve 81. The pressure relief valve 81 is a removable flap that is located against the manifold 82 of the cannula. The relief valve 81 is preferably made from a soft flexible material, such as silicone. The manifold 82 has three recesses 83, 84, 85 located in it. In use, the middle 84 of these recesses has located within it an arrow shaped protrusion 86 that forms part of the relief valve 81. The protrusion 86, being made of a soft flexible material can be pushed through recess 84, in order to locate the valve 81 in the manifold. The shape of the protrusion is such that a large pulling force on the valve 81 is required before the protrusion 84 can be removed from the manifold 82. As the ends 87, 88 of the flap 81 are made from a supple material they are capable of being pushed outwards (as shown in Figure 9), when the cannula is in use and when excessive pressure exists in the manifold or prongs, to allow gases to be released through the side recesses 83, 85 on the manifold.

It must be appreciated that Figures 8 and 9 merely show one embodiment of nasal cannula with a pressure relief valve. Other embodiments with pressure relief valves, for example, embodiments including pressure relief valves at the humidification means, humidified gases transport means or elsewhere on the nasal cannula are envisaged.

The cannula of the present invention does not require rebreathing of expired gases; rather the cannula reduces anatomical dead space by flushing the pharynx with fresh respiratory gases. Further, the nasal cannula of the present invention does not deliver continuous positive airway pressure, but instead delivers a form of bi-level positive airway pressure in which PEEP is greater than IPAP.

A further consequence that is provided by the nasal cannula in allowing for the exceeding of peak inspiratory flow is that all gases the patient is breathing are being delivered from the cannula and do not contain any portion of room air as in the prior art. This allows the oxygen percentage in the patient's breath to be controlled over the full range (up to 100%) to be known. This has previously only been possible with a mask, which is much more claustrophobic and restrictive to the patient and severely hinders the patients' ability to talk or eat. The cannula, therefore, incorporates the advantages of Venturi mask, nasal cannula, and non-rebreathing mask as stated earlier without the disadvantages of discomfort (Venturi mask), inconsistent Fi02 (prior art nasal cannula), need for a tight seal between mask and patient (non-rebreathing mask), and possibility of rebreathing expired gases (non-rebreathing mask).

Finally, in summary, the nasal cannula of the present invention allows for the delivery of humidified air (whether the air is blended with or without oxygen) to a patient at flows greater than 30 L/min that offers the following benefits over standard cannula.
1) High FiO₂ (fraction of inspired oxygen), where normal cannula provide less than 32%.
2) Known FiO₂ that is equivalent to a facemask.
3) PEEP (positive end expiratory pressure) caused by breathing out against the inward flow of gases.
4) IPAP (inspiratory positive airway pressure) where delivered flow is greater than the peak inspiratory flow.
5) Delivers pressure oscillations.
6) Reduces breathlessness.

### Statements of embodiments

1. A breathing assistance apparatus comprising:
   a pressurised source of gases,
   humidification means adapted to, in use, be in fluid communication with said source of gases and adapted to in use humidify said gases,
   humidified gases transport means adapted to, in use, be in fluid communication with said humidification means and adapted to in use convey said humidified gases,
   heating means disposed within said transport means and adapted to in use heat said gases as they pass through said transport means, and
   nasal cannula, that are adapted to deliver said humidified gases to said patient, said nasal cannula including at least one wide bore conduit that allows high flow delivery of said humidified gases and creates positive airway pressure in said patient's airway, said nasal cannula adapted to, in use, be in fluid communication with said transport means.
2. A breathing assistance apparatus according to clause 1 wherein said cannula is provided with two nasal prongs that do not seal within the nares of said patient.
3. A breathing assistance apparatus according to clause 2 wherein said nasal prongs are shaped so they are anatomically correct to said patient's nasal passage.
4. A breathing assistance apparatus according to any one of clauses 2 or 3 wherein said nasal prongs are angled inwards toward the septum of the nose and are round or elliptical in shape, or in a shape that corresponds to the inner dimensions of said patient's nares.
5. A breathing assistance apparatus according to any one of clauses 2 to 4 wherein each of said nasal prongs directs gases flow toward the back of the patient's head, so that the gases flow directly through said patient's nasal passages.
6. A breathing assistance apparatus according to any one of claims 1 to 5 wherein one of said humidification means, said humidified gases transport means and said nasal cannula includes a pressure relief valve to ensure gases pressure can be released upon improper fitting of said prongs or sealing of said prongs within said patient's nares.
7. A breathing assistance apparatus according to any one of clauses 1 to 6 wherein a significant portion of said gases comprise oxygen gas.
8. A breathing assistance apparatus according to clause 7 wherein said portion is greater then 21%.
9. A breathing assistance apparatus according to clause 1 wherein said heating means comprises a heater wire disposed within, throughout or around said transport means.
10. A breathing assistance apparatus according to any one of clauses 1 or 9 wherein said heating means is adapted to maintain said gases at a temperature above the saturation point.

## Claims

1. A nasal cannula (30, 70) comprising:
a single inlet provided by one inlet tube (71) adapted to, in use, be in fluid communication with a pressurised source of gases, and
nasal prongs (40, 41; 72, 73) adapted to, in use, be in fluid communication with said one inlet tube (71) and adapted to, in use, deliver said gases to a patient,
wherein said one inlet tube (71) is adapted to, in use, allow said gases to flow through said nasal prongs (40, 41; 72, 73) and into the corresponding nares of the patient and,
said nasal cannula (30, 70) attached to a body section (44, 74), wherein said nasal cannula (70) and said body section (44, 74) are maintained against the patient's face by way of a head strap (75), said body section (44, 74) shaped so as to fit the contours of the patient's face,
**characterised in that** said nasal prongs are, in use, angled inwards towards the septum of the nose and are curved towards the sagittal (midline) plane and,
wherein said nasal prongs, in use, follow the nasal cavity towards the posterior and follow the inner shape of the patient's nostrils and, wherein the top (45, 46) of said nasal prongs point, in use, towards the back of the patient's head.

2. A nasal cannula as claimed in claim 1, wherein said nasal prongs (40, 41; 72, 73) are attached to said one inlet tube (71).

3. A nasal cannula as claimed in claim 1 or claim 2, wherein said one inlet tube (71) extends to one side of said nasal cannula (30, 70).

4. A nasal cannula as claimed in any one of claims 1 to 3, wherein said nasal prongs (40, 41; 72, 73) do not create a seal against the nares of the patient's nose.

5. A nasal cannula as claimed in any one of claims 1 to 4, wherein said nasal cannula (30, 70) comprises a pressure relief valve (81).

6. A nasal cannula as claimed in any one of claims 1 to 5, wherein an entire assembly of the nasal cannula, including the nasal cannula (7), body section (74) and nasal prongs (40, 41; 72, 73) attaching the nasal cannula (30, 70) to the patient's face and nares, are moulded from silicone or other flexible material.

7. A breathing assistance apparatus comprising:
a pressurised source of gases,
humidification means (1) adapted to, in use, be in fluid communication with said pressurised source of gases and adapted to in use humidify said gases,
gases transport means (6) adapted to, in use, be in fluid communication with said humidification means (1) and adapted to in use convey said gases,
heating means (10) disposed within said transport means (6) and adapted to in use heat said gases as they pass through said transport means (6), and
nasal cannula (30, 70) as defined in any one of claims 1 to 6, wherein, in use, said nasal cannula allows said gases to flow through said nasal prongs (40, 41; 72, 73) and into the corresponding nares of the patient.

## Patentansprüche

1. Nasenkanüle (30, 70) umfassend:
einen einzigen Einlass, bereitgestellt von einem Einlassschlauch (71), der ausgelegt ist, um bei Gebrauch in Fluidverbindung mit einer druckbeaufschlagten Gasquelle zu stehen, und
Nasengabeln (40, 41; 72, 73) die ausgelegt sind, um bei Gebrauch in Fluidverbindung mit besagtem einem Einlassschlauch (71) zu stehen, und ausgelegt sind, um bei Gebrauch einem Patienten besagte Gase zuzuführen,
worin besagter einer Einlassschlauch (71) ausgelegt ist, um es besagten Gasen bei Gebrauch zu ermöglichen, durch besagte Nasengabeln (40, 41; 72, 73) und in die korrespondierenden Nasenlöcher des Patienten zu strömen, und
besagte Nasenkanüle (30, 70) an einem Körperabschnitt (44, 74) angebracht ist, worin besagte Nasenkanüle (70) und besagter Körperabschnitt (44, 74) mittels eines Kopfbands (75) auf das Gesicht des Patienten gehalten werden, wobei besagter Körperabschnitt (44, 74) so geformt ist, dass er sich den Gesichtskonturen des Patienten anpasst,
**dadurch gekennzeichnet, dass** besagte Nasengabeln bei Gebrauch nach innen hin zum Septum der Nase angewinkelt sind und hin zur sagittalen (Mittelinien-) Ebene gekrümmt sind, und
worin besagte Nasengabeln, bei Gebrauch, dem Nasenhohlraum hin zur Rückseite folgen und der inneren Form der Nasenöffnungen des Patienten folgen, und
worin die Oberseiten (45, 46) besagter Nasengabeln bei Gebrauch hin zum Hinterkopf des Patienten zeigen.

2. Nasenkanüle nach Anspruch 1, worin besagte Nasengabeln (40, 41; 72, 73) an besagtem einem Einlassschlauch (71) angebracht sind.

3. Nasenkanüle nach Anspruch 1 oder Anspruch 2, worin sich besagter einer Einlassschlauch (71) zu einer Seite von besagter Nasenkanüle (30, 70) erstreckt.

4. Nasenkanüle nach irgendeinem Anspruch 1 bis 3, worin besagte Nasengabeln (40, 41; 72, 73) keine Dichtung gegen die Nasenlöcher der Nase des Patienten herstellen.

5. Nasenkanüle nach irgendeinem Anspruch 1 bis 4, worin besagte Nasenkanüle (30, 70) ein Druckentlastungsventil (81) umfasst.

6. Nasenkanüle nach irgendeinem Anspruch 1 bis 5, worin eine gesamte Anordnung der Nasenkanüle einschließlich der Nasenkanüle (7), des Körperabschnitts (74) und der Nasengabeln (40, 41; 72, 73) zum Anbringen der Nasenkanüle (30, 70) am Gesicht und an den Nasenlöchern des Patienten aus Silikon oder einem anderen flexiblen Material geformt ist.

7. Gerät zur Unterstützung der Atmung, umfassend:
eine druckbeaufschlagte Gasquelle,
Befeuchtungsmittel (1), das ausgelegt ist, um bei Gebrauch in Fluidverbindung mit besagter druckbeaufschlagter Gasquelle zu stehen, und ausgelegt ist, um besagte Gase bei Gebrauch zu befeuchten,
Gastransportmittel (6), das ausgelegt ist, um bei Gebrauch in Fluidverbindung mit besagtem Befeuchtungsmittel (1) zu stehen, und ausgelegt ist, um besagte Gase bei Gebrauch zu befördern,
Erwärmungsmittel (10), das innerhalb von besagtem Transportmittel (6) angeordnet und ausgelegt ist, um besagte Gase bei Gebrauch zu erwärmen, während sie besagtes Transportmittel (6) durchlaufen, und
Nasenkanüle (30, 70) wie in irgendeinem Anspruch 1 bis 6 definiert, worin, bei Gebrauch, besagte Nasenkanüle es besagten Gasen erlaubt, durch besagte Nasengabeln (40, 41; 72, 73) und in die korrespondierenden Nasenlöcher des Patienten zu strömen.

## Revendications

1. Canule nasale (30, 70) comprenant :
une entrée unique fournie par un tube d'entrée (71) conçu pour, lors de l'utilisation, être en communication fluidique avec une source pressurisée de gaz ; et
des pinces nasales (40, 41 ; 72, 73) conçues pour, lors de l'utilisation, être en communication fluidique avec ledit tube d'entrée (71), et conçues pour, lors de l'utilisation, distribuer lesdits gaz à un patient,
ledit tube d'entrée (71) étant conçu pour, lors de l'utilisation, permettre l'écoulement desdits gaz à travers lesdites pinces nasales (40, 41 ; 72, 73) et dans les narines correspondantes du patient, et
ladite canule nasale (30, 70) étant fixée à une partie de corps (44, 74), ladite canule nasale (70) et ladite partie de corps (44, 74) étant maintenues contre le visage du patient au moyen d'une bride (75), ladite partie de corps (44, 74) présentant une forme s'adaptant aux contours du visage du patient,
**caractérisée en ce que** les pinces nasales sont, lors de l'utilisation, orientées vers l'intérieur vers la cloison nasale et recourbées vers le plan sagittal (médian), et
lesdites pinces nasales, lors de l'utilisation, suivant la cavité nasale vers l'arrière et suivant la forme intérieure des narines du patient, et
le haut (45, 46) desdites pinces nasales étant dirigé, lors de l'utilisation, vers l'arrière de la tête du patient.

2. Canule nasale selon la revendication 1, dans laquelle lesdites pinces nasales (40, 41 ; 72, 73) sont fixées audit tube d'entrée (71).

3. Canule nasale selon la revendication 1 ou 2, dans laquelle ledit tube d'entrée (71) s'étend vers un côté de ladite canule nasale (30, 70).

4. Canule nasale selon l'une quelconque des revendications 1 à 3, dans laquelle lesdites pinces nasales (40, 41 ; 72, 73) ne créent pas de joint contre les narines du nez du patient.

5. Canule nasale selon l'une quelconque des revendications 1 à 4, ladite canule nasale (30, 70) comprenant une soupape de surpression (81).

6. Canule nasale selon l'une quelconque des revendications 1 à 5, dans laquelle un ensemble entier de la canule nasale, incluant la canule nasale (7), la partie de corps (74) et les pinces nasales (40, 41 ; 72, 73) fixant la canule nasale (30, 70) au visage et aux narines du patient, est moulé en silicone ou dans un autre matériau souple.

7. Appareil d'assistance respiratoire, comprenant :
une source pressurisée de gaz ;
un moyen d'humidification (1) conçu pour, lors de l'utilisation, être en communication fluidique ladite source pressurisée de gaz, et conçu pour, lors de l'utilisation, humidifier lesdits gaz ;
un moyen de transport de gaz (6) conçu pour, lors de l'utilisation, être en communication fluidique ledit moyen d'humidification (1), et conçu pour, lors de l'utilisation, transporter lesdits gaz ;
un moyen de chauffage (10) disposé entre ledit moyen de transport (6), et conçu pour, lors de l'utilisation, chauffer lesdits gaz lorsqu'ils passent dans ledit moyen de transport (6) ; et
une canule nasale (30, 70) selon l'une quelconque des revendications 1 à 6, ladite canule nasale permettant, lors de l'utilisation, l'écoulement desdits gaz à travers lesdites pinces nasales (40, 41 ; 72, 73) et dans les narines correspondantes du patient.
